# EUROPÄISCHE PATENTSCHRIFT

(11) **EP 2 030 605 B1**
(45) Veröffentlichungstag und Bekanntmachung des Hinweises auf die Patenterteilung: **01.02.2012**
(21) Anmeldenummer: 08159386.5
(22) Anmeldetag: 01.07.2008
(51) Int. Cl.: A61K 8/06, A61K 8/89, C08G 77/38, C11D 1/82, C11D 3/37

(54) **Emulgator-Systeme enthaltend estermodifizierte Organopolysiloxane und deren Verwendung zur Herstellung kosmetischer oder pharmazeutischer Kompositionen**
Emulsifier systems comprising ester-modified organopolysiloxanes and their use for producing cosmetic or pharmaceutical compositions
Systèmes émulsifiants comprenant de organopolysiloxanes modifiés par ester et leur utilisation pour la fabrication de compositions cosmétiques ou pharmaceutiques

(30) Priorität: 29.08.2007 DE 102007041028
(43) Veröffentlichungstag der Anmeldung: 04.03.2009
(73) Patentinhaber: Evonik Goldschmidt GmbH, 45127 Essen (DE)
(72) Erfinder: Thum, Oliver, 40880 Ratingen (DE); Ferenz, Michael, 45147 Essen (DE); Hartung, Christian, 45133 Essen (DE); Meyer, Jürgen, 45134 Essen (DE)

(56) Entgegenhaltungen:
- EP-A- 1 816 154
- US-A- 5 226 923
- US-A- 5 306 838
- "Römpp Lexikon Chemie, 10. Auflage" 1997, GEORG THIEME VERLAG , STUTTGART, NEW YORK , XP002508368 * Seite 1149 - Seite 1150 *

## Beschreibung

Die Erfindung betrifft Emulgator-Systeme, die estermodifizierte Organopolysiloxane der allgemeinen Formel (I) umfassen, sowie kosmetische, dermatologische oder pharmazeutische Formulierungen, die diese Emulgator-Systeme enthalten.

### Stand der Technik:

Die Erfindung betrifft die Verwendung estermodifizierter Organopolysiloxane zur Herstellung kosmetischer oder pharmazeutischer Emulsionen oder Dispersionen.

Organomodifizierte Siloxane werden in den verschiedensten Applikationen eingesetzt. Ihre Eigenschaften lassen sich durch die Art der Modifikation, sowie durch die Modifikationsdichte gezielt einstellen.

So können zum Beispiel mit Allylpolyethern organophile oder nichtionische hydrophile Gruppen an ein Siloxangerüst gebunden werden. Derartige Verbindungen finden ihren Einsatz zum Beispiel als Polyurethan-Schaum-Stabilisatoren, als Entschäumer in Treibstoffen oder als Additive in Farben und Lacken.

Durch Umsetzung mit α-Olefinen wird dagegen das Siloxan mit hydrophoben Gruppen verknüpft. Die so erhaltenen Siliconwachse dienen zum Beispiel als Additiv in Personal-Care-Applikationen.

Es zeigt sich in vielen Anwendungsgebieten, dass die Wirkung des Siloxans entscheidend von der Verträglichkeit mit der entsprechenden Formulierung abhängt.

Als kosmetischer Emulgator geeignet sind zum Beispiel Siloxane, die neben aliphatischen Gruppen auf Basis von α-Olefinen Polyether tragen. Beispielsweise das Verkaufsprodukt ABIL EM 90 der Goldschmidt GmbH, Deutschland.

Da polyetherhaltige Verbindungen in letzter Zeit zunehmend in Kritik geraten sind, besteht Bedarf an siloxanbasierten Emulgatoren, die keine Polyethergruppen tragen, gleichzeitig aber über gute Emulgiereigenschaften verfügen.

Eine Aufgabe der vorliegenden Erfindung bestand daher darin, neuartige, polyetherfreie, organomodifizierte Siloxane zu entwickeln, die als leistungsfähige Emulgatoren eingesetzt werden können.

### Beschreibung der Erfindung:

Überraschenderweise wurde gefunden, dass estermodifizierte Organopolysiloxane der allgemeinen Formel I leistungsfähige Emulgatoren darstellen.

Gegenstand der Erfindung sind daher Emulgator-Systeme, die aus estermodifizierten Organopolysiloxanen der allgemeinen Formel (I) und mindestens einem Co-Emulgator bestehen.

Ein Vorteil dieser Emulgator-Systeme ist, dass natürlich vorkommende Fettsäuren und somit nachwachsende Rohstoffe eingesetzt werden können; diese bieten einen günstigen Zugang zu nachwachsenden hydrophoben Komponenten die bislang in siloxanbasierten Emulgatoren keine Verwendung fanden. Weiterhin bietet die breite Palette kommerziell verfügbarer Fettsäuren darüber hinaus die Möglichkeit, durch sorgfältige Auswahl der Acylreste die Eigenschaften der erfindungsgemäßen Emulgatoren weiter feinzujustieren.

Die erfindungsgemäßen Emulgator-Systeme werden nachfolgend beispielhaft beschrieben, ohne dass die Erfindung auf diese beispielhaften Ausführungsformen beschränkt sein soll. Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Werden im Rahmen der vorliegenden Erfindung Verbindungen, wie z. B. organomodifizierte Polysiloxane, beschrieben, die verschiedene Einheiten mehrfach aufweisen können, so können diese statistisch verteilt (statistisches Oligomer) oder geordnet (Blockoligomer) in diesen Verbindungen vorkommen. Angaben zu Anzahl von Einheiten in solchen Verbindungen sind als Mittelwert, gemittelt über alle entsprechenden Verbindungen zu verstehen. Als Emulgator-System ist im Rahmen dieser Erfindung ein Emulgator zu verstehen, der mindestens aus einer Substanz der allgemeinen Formel (I) und mindestens einem Co-Emulgator besteht.

Gegenstand der Erfindung sind Emulgator-Systeme enthaltend die mit organischen Estern modifizierte Siloxane der allgemeinen Formel (I) 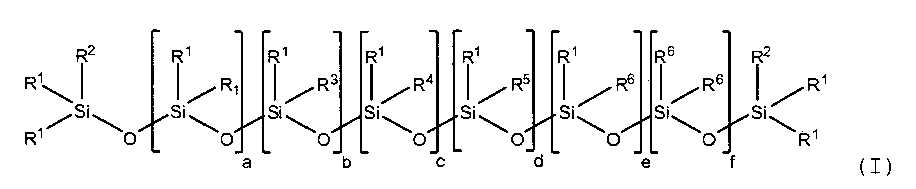 wobei
N a + b + c + d + e + f + 2 = 20 bis 250, vorzugsweise 50 bis 150,
a 1 bis 230, vorzugsweise 10 bis 130,
b 1 bis 100, vorzugsweise 5 bis 30,
c 1 bis 100, vorzugsweise 5 bis 30,
d 0 bis 50, vorzugsweise 0,
e 0 bis 10, vorzugsweise 0,
f 0 bis 10, vorzugsweise 0,
R¹ unabhängig voneinander gleich oder verschieden und aus folgender Gruppe: gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen, vorzugsweise Alkylgruppen mit 1 bis 4 C-Atomen oder Phenyl, insbesondere Methyl,
R² unabhängig voneinander gleich oder verschieden und aus folgender Gruppe: R¹, R³, R⁴, oder R⁵, bevorzugt R¹,
R³ unabhängig voneinander gleiche oder verschiedene Esterreste der allgemeinen Formel (Ia) 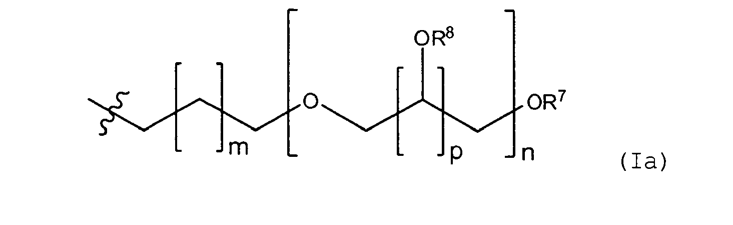 wobei
m 1 bis 4, bevorzugt 1 oder 4,
n 0 oder 1,
p 0 oder 1, bevorzugt 0,
R⁷ die Acylreste einbasischer, gesättigter oder ungesättigter, linearer oder verzweigter Fettsäuren mit 6 bis 30 Kohlenstoffatomen, insbesondere mit 8 bis 22 Kohlenstoffatomen,
R⁸ Wasserstoff oder R⁷, bevorzugt Wasserstoff,
R⁴ die Reste der allgemeinen Formel (Ib) 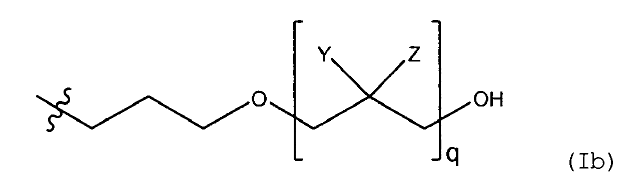 wobei
q 1 bis 20,
Y und Z unabhängig voneinander gleiche oder verschiedene Reste aus der Gruppe -H, -OH, -CH₃, -CH₂CH₃ oder -CH₂OH, sind, wobei mindestens ein Rest aus der Gruppe -OH oder -CH₂OH sein muss,
R⁵ unabhängig voneinander gesättigte oder ungesättigte, gegebenenfalls verzweigte Alkylgruppen mit 1 bis 30 C-Atomen oder Alkarylreste mit 7 bis 30 C-Atomen, vorzugsweise Alkylgruppen mit 6 bis 22 C-Atomen,
R⁶ unabhängig voneinander gleiche oder verschiedene Reste der allgemeinen Formel (Ic) 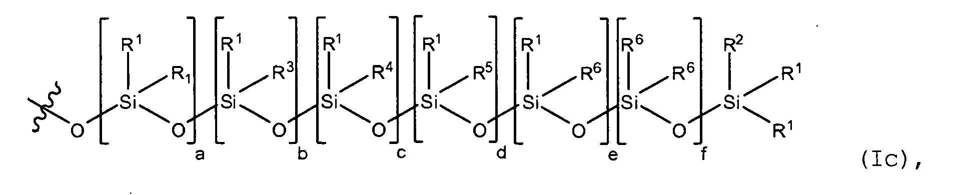 wobei a, b, c, d, e, f, R¹ R², R³, R⁴, R⁵ und R⁶ wie oben definiert sind.

In einer bevorzugten Ausführungsform ist m gleich 1 und n gleich 1. In einer weiteren bevorzugten Ausführungsform ist m gleich 4 und n gleich 0.

Reste gemäß der allgemeinen Formel (Ib) können insbesondere Verbindungen mit
q 1,
Y -OH und
Z -H, oder

q 1,
Y -CH₂OH und
Z -CH₂CH₃, oder

q 2 bis 20, bevorzugt 3 bis 10,
Y -OH und
Z -H, sein.

Hierbei ist dem Fachmann geläufig, dass es sich in letzterem Fall um vom Polyglycerin abgeleitete Verbindungen handelt, die auch andere, als die in Formel (Ib) angegebene 1,3-Verknüpfungen aufweisen können. Dabei kann es sich beispielsweise um technische Polyglycerinmischungen handeln, die z. B. durch alkalisch katalysierte Kondensation von Glycerin bei erhöhten Temperaturen erhalten werden können, aus denen sich gegebenenfalls durch Destillationsverfahren Fraktionen mit dem erwünschten Kondensationsgrad erhalten lassen können.

Ebenfalls können auch Polyglycerine, die auf anderem Wege, z. B. aus Epichlorhydrin, Glycidol oder Glycerin gewonnen werden können und von den Firmen Daicel oder Solvay vertrieben werden, eingesetzt werden.

Bei dem als Rest R⁷ eingesetzten Acylresten können natürliche Fettsäuren auf Basis natürlicher pflanzlicher oder tierischer Öle eingesetzt werden. Bevorzugt werden natürliche Fettsäuren wie z.B. Capronsäure, Caprylsäure, Caprinsäure, Laurinsäure, Myristinsäure, Palmitinsäure, Palmitoleinsäure, Isostearinsäure, Stearinsäure, 12-Hydroxystearinsäure, Dihydroxystearinsäure, Ölsäure, Linolsäure, Petroselinsäure, Elaidinsäure, Arachinsäure, Behensäure, Erucasäure, Gadoleinsäure, Linolensäure, Eicosapentaensäure, Docosahexaensäure oder Arachidonsäure, allein oder in Mischung eingesetzt. Der Rest R⁷ kann ebenfalls der Acylrest von Polykondensationsprodukten von hydroxyfunktionalisierten Säuren, beispielsweise Poly-12-hydroxystearinsäure oder Polyricinolsäure sein. Bei dem als Rest R⁷ eingesetzten Acylresten kann es sich um technische Mischungen handeln, beispielsweise um Mischungen von natürlichen Fettsäuren, z. B. Rapsölfettsäure, Sojaölfettsäure, Sonnenblumenölfettsäure, Talgölfettsäure, Palmölfettsäure, Palmkernölfettsäure, Kokosfettsäure, die in Anhängigkeit von ihrer konkreten Quelle und den eingesetzten Aufreinigungsverfahren in ihrer genauen Zusammensetzung Schwankungen unterworfen sein können, sowie typische Nebenbestandteile, wie ungesättigte, funktionalisierte oder verzweigte Komponenten enthalten können. Darüber hinaus können auch Mischungen von Säuren anderer Herkunft, zum Beispiel auf Basis petrochemischer Verfahren, eingesetzt werden.

Weitere bevorzugte Ausführungsformen sind Verbindungen, bei denen:
N 80 bis 120, bevorzugt 90 bis 110, insbesondere 100 und
a 60 bis 100, bevorzugt 65 bis 85, insbesondere 73 und
b 10 bis 30, bevorzugt 12 bis 20, insbesondere 16 und
c 5 bis 20, bevorzugt 8 bis 15, insbesondere 9 und
d = e = f 0 und
R¹ = R² CH₃ und
m 1 und
n 1 und
p 0 und
R⁷ der Acylrest einer Fettsäure mit 8 bis 18 C-Atomen, bevorzugt mit 12 bis 14 C-Atomen, oder einer Mischung daraus, insbesondere der Acylrest einer gehärteten Kokosfettsäure, also eine technische Mischung aus vorwiegend Laurinsäure und Myristinsäure und
q 1 und
Y OH und
Z H,
   oder
N 80 bis 120, bevorzugt 90 bis 110, insbesondere 100 und
   a 60 bis 100, bevorzugt 65 bis 85, insbesondere 73 und
   b 10 bis 30, bevorzugt 12 bis 20, insbesondere 16 und
   c 5 bis 20, bevorzugt 8 bis 15, insbesondere 9 und d = e = f 0 und
   R¹ = R² CH₃ und
   m 1 und
   n 1 und
   p 1 und
   R⁷ der Acylrest einer Fettsäure mit 8 bis 18 C-Atomen, bevorzugt mit 12 bis 14 C-Atomen, oder einer Mischung daraus, insbesondere der Acylrest einer gehärteten Kokosfettsäure und
   R⁸ H und
   q 1 und
   Y OH und
   Z H,
   oder
N 80 bis 120, bevorzugt 90 bis 110, insbesondere 100 und
   a 60 bis 100, bevorzugt 65 bis 85, insbesondere 73 und
   b 10 bis 30, bevorzugt 12 bis 20, insbesondere 16 und
   c 5 bis 20, bevorzugt 8 bis 15, insbesondere 9 und d = e = f 0 und
   R¹ R² = CH₃ und
   m 4 und
   n 0 und
   p 0 und
   R⁷ der Acylrest einer Fettsäure mit 8 bis 18 C-Atomen, bevorzugt mit 12 bis 14 C-Atomen, oder einer Mischung daraus, insbesondere der Acylrest einer gehärteten Kokosfettsäure und
   q 1 und
   Y OH und
   Z H,
   ist.

Die Herstellung der mit organischen Estern modifizierten Siloxane kann durch Hydrosilylierung erfolgen. Die zur Hydrosilylierung eingesetzten terminal ungesättigten Ester können durch Ver- oder Umesterung der entsprechenden Alkohole mit Säuren erhalten werden, wie beispielsweise in der Patentanmeldung DE 10 2006 005100.9 dargelegt. Die zur Hydrosilylierung eingesetzten SiH-funktionellen Siloxane sind durch die dem Fachmann bekannten Verfahren der Equilibrierung, wie zum Beispiel in US 7,196,153 B2 beschrieben, erhältlich.

Die Hydrosilylierung kann nach etablierten Methoden in Gegenwart eines Katalysators durchgeführt werden. Dabei können beispielsweise Katalysatoren eingesetzt werden wie Platin-, Rhodium-, Osmium-, Ruthenium-, Palladium-, Iridium-Komplexe oder ähnliche Verbindungen bzw. die entsprechenden reinen Elemente oder deren auf Silica, Aluminiumoxid oder Aktivkohle oder ähnlichen Trägermaterialien immobilisierten Derivaten. Die Hydrosilylierung kann in Gegenwart von Pt-Katalyatoren wie Cis-Platin oder Karstedt-Katalysator [Tris(divinyltetramethyldisiloxan)bis-platin] durchgeführt werden. Die eingesetzte Menge an Katalysator kann 10⁻⁷ bis 10⁻¹ mol pro mol Olefin, bevorzugt 1 bis 20 ppm betragen. Die Hydrosilylierung kann bei Temperaturen zwischen 0 und 200 °C, bevorzugt zwischen 50 und 140 °C, durchgeführt werden. Die Reaktion kann in geeigneten Lösungsmitteln wie aliphatischen oder aromatischen Kohlenwasserstoffen, cyclischen Oligosiloxanen, Alkoholen oder Estern durchgeführt werden. Es kann auch auf den Einsatz eines Lösungsmittels verzichtet werden.

Die erfindungsgemäßen Emulgator-Systeme werden vorzugsweise als Wasser-in-Öl-, Öl-in-Wasser- oder Wasser-in-Silicon-Emulgatoren oder Dispergierhilfsmittel verwendet. Somit sind die mit Hilfe der erfindungsgemäßen Emulgator-Systeme erhaltenen Wasser-in-Öl-, Öl-in-Wasser- und Wasser-in-Silicon-Emulsionen und Dispersionen ebenfalls Gegenstand der Erfindung.

Ein weiterer Gegenstand der Erfindung ist die Verwendung der erfindungsgemäßen Emulgator-Systeme zur Herstellung kosmetischer, dermatologischer oder pharmazeutischer Formulierungen. Somit sind die kosmetischen, dermatologischen oder pharmazeutischen Formulierungen ebenfalls Gegenstand der Erfindung.

Ein weiterer Gegenstand der Erfindung ist die Verwendung der Emulgator-Systeme zur Herstellung gegebenenfalls dispergierter Feststoffe enthaltender Pflege- und Reinigungsmittel für Haushalt oder Industrie, insbesondere für harte Oberflächen, Leder oder Textilien. Somit sind die Pflege- und Reinigungsmittel für Haushalt oder Industrie und die Pflege- und Reinigungsmittel für harte Oberflächen, Leder oder Textilien ebenfalls Gegenstand der Erfindung.

Die kosmetischen, dermatologischen oder pharmazeutischen Formulierungen sowie die Pflege- und Reinigungsmittel können z.B. mindestens eine zusätzliche Komponenten enthalten, ausgewählt aus der Gruppe der
Emollients,
Co-Emulgatoren und Tenside,
Verdicker/Viskositätsregler/Stabilisatoren,
UV-Lichtschutzfilter,
Antioxidantien,
Hydrotrope (oder Polyole),
Feststoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
Biogene Wirkstoffe,
Pflegeadditive,
Lösungsmittel.

Als Emollients können alle kosmetischen Öle insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen eingesetzt werden. Ebenso sind die Veresterungsprodukte aliphatischer, difunktioneller Alkohole mit 2 bis 36 C-Atomen mit monofunktionellen aliphatischen Carbonsäuren mit 1 bis 22 C-Atomen einsetzbar. Des Weiteren eignen sich langkettige Arylsäureester wie z.B. Ester der Benzoesäure, z.B. Benzoesäureester von linearen oder verzweigten, gesättigten oder ungesättigten Alkoholen mit 1 bis 22 C-Atomen, oder auch Benzoesäureisostearylester oder Benzoesäureoctyldocecylester. Weitere als Emollients und Ölkomponenten geeignete Monoester sind z.B. die Methylester und Isopropylester von Fettsäuren mit 12 bis 22 C-Atomen wie z.B. Methyllaurat, Methylstearat, Methyloleat, Methylerucat, Isopropylpalmitat, Isopropylmyristat, Isopropylstearat, Isopropyloleat. Andere geeignete Monoester sind z.B. n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, z.B. Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie z.B. im Jojobaöl oder im Spermöl vorliegen. Geeignete Dicarbonsäureester sind z.B. Di-n-butyl-adipat, Di-n-butyl-sebacat, Di-(2-ethylhexyl)-adipat, Di-(2-hexyldecyl)-succinat, D-isotridecylacelaat. Geeignete Diolester sind z.B. Ethylenglycoldioleat, Ethylenglycol-di-isotridecanoat, Propylenglycol-di-(2-ethylhexanoat), Butandiol-di-isostearat, Butandiol-di-caprylat/caprat und Neopentylglycol-di-caprylat. Weitere Fettsäureester, die als Emollients eingesetzt werden können, sind z.B. C₁₂-₁₅ Alkylbenzoat, Dicaprylylcarbonat, Diethylhexylcarbonat. Ebenso als Emollients und Ölkomponente können längerkettige Triglyceride, d.h. dreifache Ester des Glycerins mit drei Säuremolekülen, wovon mindestens eine längerkettig ist, eingesetzt werden. Hier seien beispielhaft Fettsäuretriglyceride erwähnt; als solche können beispielsweise natürliche, pflanzliche Öle, z.B. Olivenöl, Sonnenblumenöl, Sojaöl, Erdnußöl, Rapsöl, Mandelöl, Sesamöl, Avocadoöl, Rizinusöl, Kakaobutter, Palmöl aber auch die flüssigen Anteile des Kokosöls oder des Palmkernöls sowie tierische Öle wie z.B. Klauenöl, die flüssigen Anteile des Rindertalgs oder auch synthetische Triglyceride von Capryl-Caprinsäure-Gemischen, Triglyceride aus technischer Ölsäure, Triglyceride mit Isostearinsäure, oder aus Palmitinsäure-Ölsäure-Gemischen als Emollients und Ölkomponenten eingesetzt werden. Weiterhin können Kohlenwasserstoffe, insbesondere auch flüssige Paraffine und Isoparaffine eingesetzt werden. Beispiele für einsetzbare Kohlenwasserstoffe sind Paraffinöl, Isohexadecan, Polydecen, Vaseline, Paraffinum perliquidum, Squalan, Ceresin. Weiterhin sind auch lineare oder verzweigte Fettalkohole wie Oleylalkohol oder Octyldodecanol, sowie Fettalkoholether wie Dicaprylyl Ether einsetzbar. Geeignete Siliconöle und -wachse sind z.B. Polydimethylsiloxane, Cyclomethylsiloxane, sowie aryl- oder alkyl- oder alkoxysubstituierte Polymethylsiloxane oder Cyclomethylsiloxane. Als Co-Emulgatoren oder Tenside können nichtionische, anionische, kationische oder amphotere Tenside eingesetzt werden.

Als nichtionogene Co-Emulgatoren oder Tenside können Verbindungen aus mindestens einer der folgenden Gruppen eingesetzt werden:
Anlagerungsprodukte von 2 bis 100 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C_{12/18}-Fettsäuremono- und -diester von Anlagerungsprodukten von 1 bis 100 Mol Ethylenoxid an Glycerin,
Glycerinmono- und -diester und Sorbitanmono- und -diester von gesättigten und ungesättigten Fettsäuren mit 6 bis 22 Kohlenstoffatomen und deren Ethylenoxidanlagerungsprodukte, Alkylmono- und -oligoglycoside mit 8 bis 22 Kohlenstoffatomen im Alkylrest und deren Ethylenoxidanlagerungsprodukte,
Anlagerungsprodukte von 2 bis 200 Mol Ethylenoxid an Ricinusöl und/oder gehärtetes Ricinusöl,
Partialester auf Basis linearer, verzweigter, ungesättigter bzw. gesättigter C₆-C₂₂-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose),
Mono-, Di- und Trialkylphosphate sowie Mono-, Di- und/oder Tri-PEG-alkylphosphate und deren Salze,
Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 4/12 oder 20/20 oder 18/18 oder 17/18 oder 15/15, Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL® EM 90 (Degussa)),
Mischester aus Pentaerythrit, Fettsäuren, Citronensäure und Fettalkohol gemäß DE 11 65 574 und/oder Mischester von Fettsäuren mit 6 bis 22 Kohlenstoffatomen, Methylglucose und Polyolen, vorzugsweise Glycerin oder Polyglycerin, Zitronensäureester wie z.B. Glyceryl Stearate Citrate, Glyceryl Oleate Citrate und Dilauryl Citrate.

Anionische Co-Emulgatoren oder Tenside können wasserlöslich machende anionische Gruppen wie z.B. eine Carboxylat-, Sulfat-, Sulfonat- oder Phosphat-Gruppe und einen lipophilen Rest enthalten. Hautverträgliche anionische Tenside sind dem Fachmann in großer Zahl bekannt und im Handel erhältlich. Dabei kann es sich um Alkylsulfate oder Alkylphosphate in Form ihrer Alkali, Ammonium- oder Alkanolammoniumsalze, Alkylethersulfate, Alkylethercarboxylate, Acylsarkosinate sowie Sulfosuccinate und Acylglutamate in Form ihrer Alkali- oder Ammoniumsalze handeln.

Auch kationische Co-Emulgatoren und Tenside können zugesetzt werden. Als solche können insbesondere quaternäre Ammoniumverbindungen, insbesondere solche, versehen mit mindestens einer linearen und/oder verzweigten, gesättigten oder ungesättigten Alkylkette mit 8 bis 22 C-Atomen, eingesetzt werden, so etwa Alkyltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid eingesetzt werden.

Weiterhin können Monoalklyamidoquats wie z.B. Palmitamidopropyltrimethylammoniumchlorid oder entsprechende Dialkylamidoquats eingesetzt werden.

Weiterhin können biologisch gut abbaubare quaternäre Esterverbindungen eingesetzt werden, bei denen es sich um quaternierte Fettsäureester auf Basis von Mono-, Di- oder Triethanolamin handeln kann. Weiterhin können Alkylguanidiniumsalze als kationische Co-Emulgatoren beigesetzt sein.

Weiterhin ist es möglich, amphotere Tenside wie z.B. Betaine, Amphoacetate oder Amphopropionate zusammen mit den erfindungsgemäßen Polyglycerinestern einzusetzen.

Als Verdicker zur Verdickung von Ölphasen kommen alle dem Fachmann bekannten Verdickungsmittel in Frage. Insbesondere sind dabei zu nennen Wachse, wie hydriertes Castorwachs, Bienenwachs oder Microwachs. Weiterhin können auch anorganische Verdickungsmittel eingesetzt werden wie Silica, Alumina oder Schichtsilikate (z.B. Hectorit, Laponit, Saponit). Diese anorganischen Ölphasenverdicker können dabei hydrophob modifiziert sein. Zur Verdickung/Stabilisierung von Wasser-in-Öl-Emulsionen können dabei insbesondere Aerosile, Schichtsilikate und /oder Metallsalze von Fettsäuren, wie z.B. Zinkstearat eingesetzt werden.

Als Viskositätsregler für wässrige Tensidsysteme können z.B. NaCl, niedermolekulare nichtionische Tenside, wie Cocoamide DEA/MEA und Laureth-3, oder polymere, hochmolekulare, assoziative, hochethoxylierte Fettderivate, wie PEG-200 Hydrogenated Glyceryl Palmate enthalten sein.

Als UV-Lichtschutzfilter können beispielsweise organische Substanzen eingesetzt werden, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche UVB-Lichtschutzfilter sind z.B. zu nennen:
3-Benzylidencampher und dessen Derivate, z.B. 3-(4-Methylbenzyliden)campher,
4-Aminobenzoesäurederivate, wie z.B. 4-(Dimethylamino)-benzoesäure-2-ethylhexylester,4-(Dimethylamino)benzoesäure-2-ethylhexylester und 4-(Dimethylamino)benzoesäureamylester Ester der Zimtsäure, wie z.B. 4-Methoxyzimtsäure-2-ethylhexylester, 4-Methoxyzimtsäureisopentylester, 2-Cyan-3-phenyl-zimtsäure-2-ethylhexylester (Octocrylene),
Ester der Salicylsäure, wie z.B. Salicylsäure-2-ethylhexylester, Salicylsäure-4-isopropylbenzylester, Salicylsäurehomomenthylester,
Derivate des Benzophenons, wie z.B. 2-Hydroxy-4-methoxybenzophenon, 2-Hydroxy-4-methoxy-4'-methylbenzophenon, 2,2'-Dihydroxy-4-methoxybenzophenon,
Ester der Benzalmalonsäure, wie z.B. 4-Methoxybenzmalonsäuredi-2-ethylhexyester,
Triazinderivate, wie z.B. 2,4,6-Trianilino-(p-carbo-2'-ethyl-1'-hexyloxy)-1,3,5-triazin und Octyltriazon, Propan-1,3-dione, wie z.B. 1-(4-tert.Butylphenyl)-3-(4'-methoxyphenyl)propan-1,3-dion.

Als wasserlösliche UVB-Lichtschutzfilter kommen in Frage:
2-Phenylbenzimidazol-5-sulfonsäure und deren Alkali-, Erdalkali-, Ammonium-, Alkylammonium-, Alkanolammonium- und Glucammoniumsalze,
Sulfonsäurederivate von Benzophenon, wie z.B. 2-Hydroxy-4-methoxybenzophenon-5-sulfonsäure und ihre Salze, Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UVA-Lichtschutzfilter kommen insbesondere Derivate des Benzoylmethans in Frage, wie beispielsweise 1-(4'-tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden.

Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, z.B. zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-16-(2H-benzotriazole-2-yl)-4-(1, 1, 3, 3-tetramethylbutyl)-phenol} mit einer Partikelgröße von < 200 nm, das z.B. als 50 %ige wässrige Dispersion erhältlich ist.

Weitere geeignete UV-Lichtschutzfilter sind der Übersicht von P. Finkel in SÖFW-Journal 122, 543 (1996) zu entnehmen. Neben den beiden vorgenannten Gruppen primärer UV-Lichtschutzfilter können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Als Antioxidantien können z.B. Superoxid-Dismutase, Tocopherole (Vitamin E), Dibutylhydroxytoluol und Ascorbinsäure (Vitamin C) eingesetzt werden.

Als Hydrotrope können zur Verbesserung des Fließverhaltens und der Anwendungseigenschaften beispielsweise Ethanol, Isopropylalkohol oder Polyole eingesetzt werden. Polyole, die hier in Betracht kommen, können 2 bis 15 Kohlenstoffatome und mindestens zwei Hydroxylgruppen besitzen. Typische Beispiele sind:
Glycerin, Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton,
technische Oligoglyceringemische mit einem Eigenkondensationsgrad von 1,5 bis 10 wie etwa technische Diglyceringemische mit einem Diglyceringehalt von 40 bis 50 Gew.-%,
Methylolverbindungen, wie insbesondere Trimethylolethan, Trimethylolpropan, Trimethylolbutan, Pentaerythrit und Dipentaerythrit,
Niedrigalkylgucoside, insbesondere solche mit 1 bis 4 Kohlenstoffatomen im Alkylrest, wie beispielsweise Methyl- und Butylglucosid,
Zuckeralkohole mit 5 bis 12 Kohlenstoffatomen, wie beispielsweise Sorbit oder Mannit,
Zucker mit 5 bis 12 Kohlenstoffatomen, wie beispielsweise Glucose oder Saccharose,
Aminozucker, wie beispielsweise Glucamin.
Als Feststoffe können beispielsweise Eisenoxidpigmente, Titandioxid oder Zinkoxidpartikel und die zusätzlich unter "UV-Schutzmittel" genannten eingesetzt werden. Weiterhin können auch Partikel eingesetzt werden, die zu speziellen sensorischen Effekten führen, wie etwa Nylon-12, Bornitrid, Polymerpartikel wie etwa Polyacrylat- oder Polymethylacrylatpartikel oder Siliconelastomere.

Als Perlglanzadditive können z.B. Glycoldistearate oder PEG-3 Distearat eingesetzt werden.

Als Deodorantwirkstoffe kommen z.B. in Frage Geruchsüberdecker wie die gängigen Parfümbestandteile, Geruchsabsorber, beispielsweise die in der Patentoffenlegungsschrift DE 40 09 347 beschriebenen Schichtsilikate, von diesen insbesondere Montmorillonit, Kaolinit, Ilit, Beidelit, Nontronit, Saponit, Hectorit, Bentonit, Smectit, ferner beispielsweise Zinksalze der Ricinolsäure. Keimhemmende Mittel sind ebenfalls geeignet, eingearbeitet zu werden. keimhemmende Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol), Ethylhexyl glycerylether, Polyglyceryl-3 caprylat (TEGO^{®} Cosmo P813, Degussa), sowie die in den Patentoffenlegungsschriften DE 198 55 934, DE 37 40 186, DE 39 38 140, DE 42 04 321, DE 42 29 707, DE 42 29 737, DE 42 38 081, DE 43 09 372, DE 43 24 219 und EP 666 732 beschriebenen wirksamen Agenzien.

Als Antitranspirantwirkstoffe können Adstringentien eingesetzt werden, beispielsweise basische Aluminiumchloride wie Aluminiumchlorhydrat ("ACH") und Aluminium-Zirkonium-Glycine-Salze ("ZAG").

Als Insekten-Repellentien können beispielsweise N,N-Diethyl-m-toluamid, 1,2-Pentandiol oder Insect Repellent 3535 eingesetzt werden.

Als Selbstbräuner können z.B. Dihydroxyaceton und Erythrulose eingesetzt werden.

Als Konservierungsstoffe können beispielsweise Mischungen einzelner oder mehrerer Alkylparabenester mit Phenoxyethanol eingesetzt werden. Bei den Alkylparabenestern kann es sich um Methlyparaben, Ethylparaben, Propylparaben und/oder Butylparaben handeln. Anstelle von Phenoxyethanol können auch andere Alkohole eingesetzt werden, wie beispielsweise Benzylalkohol oder Ethanol. Darüber hinaus können auch andere übliche Konservierungsmittel wie etwa Sorbin- oder Benzoesäure, Salicylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate, Methylisothiazolin, Chlormethyl-isothiazolin, Ethylhexylglycerin oder Caprylyl Glycol eingesetzt werden.

Als Konditioniermittel können z.B. organische quaternäre Verbindungen wie Cetrimoniumchlorid, Dicetyldimoniumchlorid, Behentrimoniumchlorid, Distearyldimoniumchlorid, Behentrimoniummethosulfat, Distearoylethyldimoniumchlorid, Palmitamidopropyltrimoniumchlorid, Guar Hydroxypropyltrimoniumchlorid, Hydroxypropylguar Hydroxypropyltrimoniumchlorid, oder Quaternium-80 oder auch Aminderivate wie z.B. Aminopropyldimethicone oder Stearamidopropyldimethylamine verwendet werden.

Als Parfüme können natürliche oder synthetische Riechstoffe oder Gemische daraus eingesetzt werden. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orange), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen z.B. die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Es können Mischungen verschiedener Riechstoffe eingesetzt werden, die gemeinsam eine ansprechende Duftnote erzeugen. Auch ätherische Öle geringer Flüchtigkeit, die meist als Aromakomponenten eingesetzt werden, eignen sich als Parfüme, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Es können Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen, eingesetzt werden.

Als Farbstoffe können die für kosmetische Zwecke geeigneten und zugelassenen Substanzen eingesetzt werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Desoxyribonucleinsäure, Coenzym Q10, Retinol, Bisabolol, Allantoin, Phytantriol, Panthenol, AHA-Säuren, Aminosäuren, Hyaluronsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte und Vitaminkomplexe zu verstehen.

Als Pflegeadditive können z.B. ethoxylierte Glycerin-Fettsäureester, wie beispielweise PEG-7 Glycerin Cocoate, oder kationische Polymere, wie beispielsweise Polyquaternium-7 oder Polyglycerinester enthalten sein.

Als Lösungsmittel können z.B. Propylenglycol, Dipropylenglycol, Glycerin, Glycerincarbonat, Wasser, Ethanol, Propanol, 1,3-Propandiol eingesetzt werden.

Bevorzugt ist die Verwendung erfindungsgemäßer Emulgator-Systeme zur Herstellung kosmetischer oder pharmazeutischer Formulierungen. Formulierungen, die durch Verwendung von Öl-in-Wasser- oder Wasser-in-Öl-Emulgatoren eine leicht streichbare Konsistenz erhalten, weil durch diese Emulgator-Systeme sich ein Öl oder ein Fett gut in eine wässrige Phase bzw. eine wässrige Phase gut in ein Öl oder ein Fett einarbeiten lässt, können beispielsweise Cremes, wie Pflegecremes, Babycremes oder Sonnenschutzcremes, Salben, Lotionen oder Schminken sein. Insbesondere kann es sich bei den kosmetischen Formulierungen auch um Formulierungen handeln, die dispergierte Feststoffe wie beispielsweise Eisenoxidpigmente, Titandioxid oder Zinkoxidpartikel enthalten. In pharmazeutischen Zubereitungen, wie Salben oder Cremes, benötigt man Öl-in-Wasser- oder Wasser-in-Öl-Emulgatoren zur Applikation von Wirkstoffen.

Erfindungsgemäße Formulierungen können daher Verwendung als ein Hautpflege-, Gesichtspflege-, Kopfpflege-, Körperpflege-, Intimpflege-, Fußpflege-, Haarpflege-, Nagelpflege, Zahnpflege- oder Mundpflegeprodukt finden. Erfindungsgemäße Formulierungen können Verwendung in Form einer Emulsion, einer Suspension, einer Lösung, einer Creme, einer Salbe, einer Paste, eines Gels, eines Öls, eines Puders, eines Aerosols, eines Stiftes, eines Sprays, eines Reinigungsproduktes, eines Schmink- oder Sonnenschutzpräparates oder eines Gesichtswassers finden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele:

### Beispiel 1:

Enzymatische Synthese von Allyloxyethanolkokosfettsäureester nach DE 10 2006 005100.9

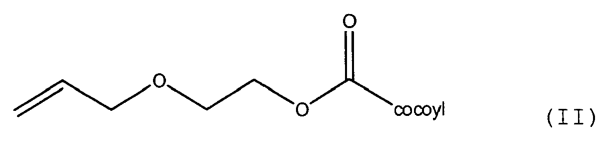

In einem Mehrhalsrundkolben wurden 134,8 g Allyloxyethanol und 243,9 g Kokosfettsäure (Edenor HK 8-18, Fa. Cognis, Monheim, Deutschland) vorgelegt und auf 40 °C erhitzt. Nach Zugabe von 18 g Novozym 435 (immobilisierte Lipase B aus *C. antarctica*, bezogen von Novozymes A/S, Bagsvaerd, Dänemark) wurde Vakuum angelegt (20 mbar) und das Reaktionswasser abdestilliert. Nach 10 Stunden wurde das immobilisierte Enzym abfiltriert. Das Filtrat lieferte 344 g Produkt ohne weitere Aufarbeitung als farblose Flüssigkeit.

### Beispiel 2:

Enzymatische Synthese von 1-Allylglyceryl-kokosfettsäureester nach DE 10 2006 005100.9

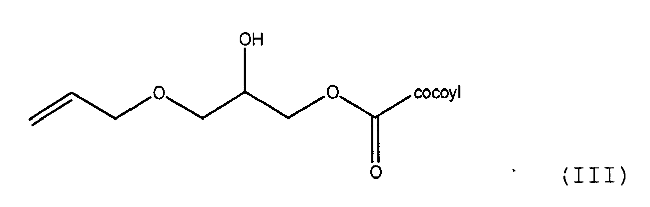

In einem Mehrhalsrundkolben wurden 185,1 g 1-Allylglycerin und 213,4 g Kokosfettsäure (Edenor HK 8-18, Fa. Cognis, Monheim, Deutschland) vorgelegt und auf 50 °C erhitzt. Nach Zugabe von 19 g Novozym 435 wurde Vakuum angelegt (20 mbar) und das Reaktionswasser abdestilliert. Nach 7 Stunden wurde das immobilisierte Enzym abfiltriert. Das Filtrat lieferte 379 g Produkt ohne weitere Aufarbeitung als farblose Flüssigkeit.

### Beispiel 3:

Enzymatische Synthese von Kokosfettsäurehexenylester nach DE 10 2006 005100.9

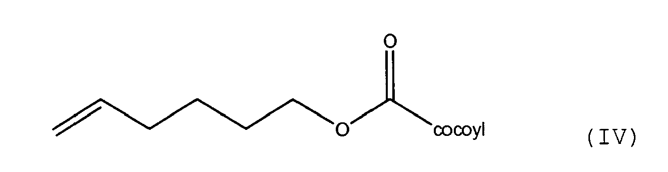

In einem Mehrhalsrundkolben wurden 140,0 g Hex-5-en-1-ol und 258,1 g Kokosfettsäure (Edenor HK 8-18, Fa. Cognis, Monheim, Deutschland) vorgelegt und auf 60 °C erhitzt. Nach Zugabe von 19 g Novozym 435 wurde Vakuum angelegt (20 mbar) und das Reaktionswasser abdestilliert. Nach 7 Stunden wurde das immobilisierte Enzym abfiltriert. Das Filtrat lieferte 360 g Produkt ohne weitere Aufarbeitung als farblose Flüssigkeit.

### Beispiel 4:

### Hydrosilylierung des Reaktionsproduktes von Beispiel 1

Herstellung eines erfindungsgemäßen Polysiloxans der allgemeinen Formel (V):

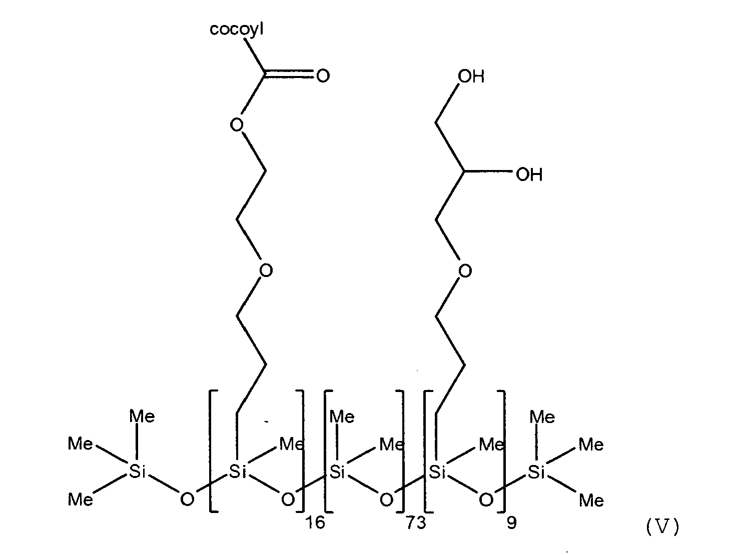

In einem Vierhalskolben, versehen mit Rührer, Tropftrichter, Thermometer und Rückflusskühler, wurden 6,2 g (47 mmol) Glycerinmonoallylether, 23,8 g (83 mmol) des Allyloxyethanolkokosfettsäureester aus Beispiel 1 und 10 ppm Karstedt-Katalysator vorgelegt und auf 95 °C erhitzt. 28,9 g (100 mmol SiH) eines SiH-Siloxans der allgemeinen Formel (VI) 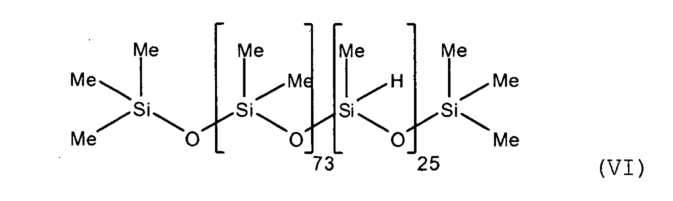 wurden zugetropft und der Ansatz für 1 h bei 95 °C gerührt. Laut SiH-Wert-Bestimmung wurde ein vollständiger Umsatz des SiH-Siloxans erhalten. Flüchtige Anteile wurden anschließend im Vakuum bei 110 °C abdestilliert. Es wurde ein viskoses, leicht trübes, fast farbloses Produkt erhalten.

### Beispiel 5:

### Hydrosilylierung des Reaktionsproduktes von Beispiel 2

Herstellung eines erfindungsgemäßen Polysiloxans der allgemeinen Formel (VII):

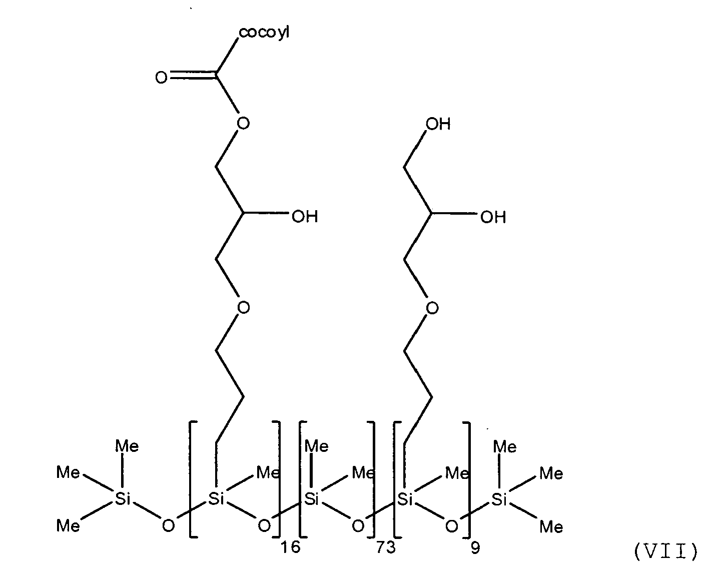

In einem Vierhalskolben, versehen mit Rührer, Tropftrichter, Thermometer und Rückflusskühler, wurden 6,2 g (47 mmol) Glycerinmonoallylether, 22,5 g (83 mmol) des 1-Allylglyceryl-kokosfettsäureester aus Beispiel 2 und 10 ppm Karstedt-Katalysator vorgelegt und auf 95 °C erhitzt. 28,9 g (100 mmol SiH) eines SiH-Siloxans der allgemeinen Formel (VI) wurden zugetropft und der Ansatz für 1 h bei 95 °C gerührt. Laut SiH-Wert-Bestimmung wurde ein vollständiger Umsatz des SiH-Siloxans erhalten. Flüchtige Anteile wurden anschließend im Vakuum bei 110 °C abdestilliert. Es wurde ein viskoses, leicht trübes, fast farbloses Produkt erhalten.

### Beispiel 6:

### Hydrosilylierung des Reaktionsproduktes von Beispiel 3

Herstellung eines erfindungsgemäßen Polysiloxans der allgemeinen Formel (VIII):

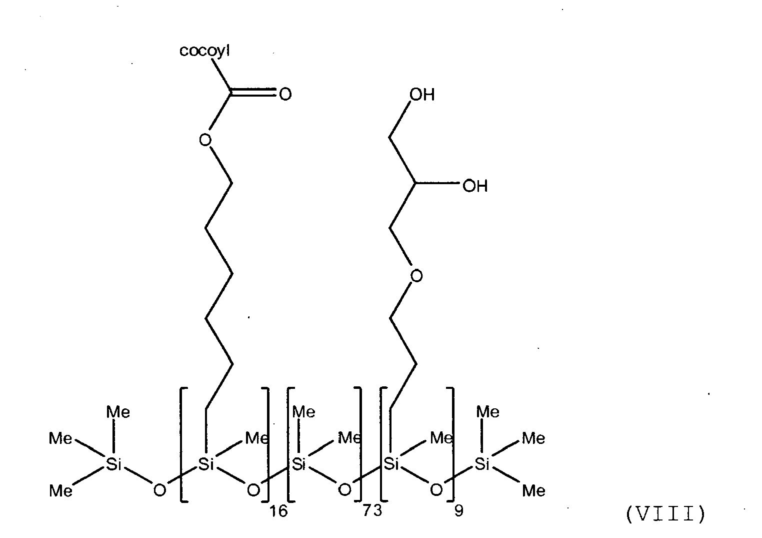

In einem Vierhalskolben, versehen mit Rührer, Tropftrichter, Thermometer und Rückflusskühler, wurden 6,2 g (47 mmol) Glycerinmonoallylether, 23,6 g (83 mmol) des Kokosfettsäurehexenylester aus Beispiel 3 und 10 ppm Karstedt-Katalysator vorgelegt und auf 95 °C erhitzt. 28,9 g (100 mmol SiH) eines SiH-Siloxans der allgemeinen Formel (VI) wurden zugetropft und der Ansatz für 1 h bei 95 °C gerührt. Laut SiH-Wert-Bestimmung wurde ein vollständiger Umsatz des SiH-Siloxans erhalten. Flüchtige Anteile wurden anschließend im Vakuum bei 110 °C abdestilliert. Es wurde ein viskoses, leicht trübes, fast farbloses Produkt erhalten.

### Anwendungsbeispiele:

Die beschriebenen kosmetischen Emulsionen sollen dazu dienen, die Verwendbarkeit der estermodifizierten Organopolysiloxane als Emulgatoren für kosmetische Emulsionen beispielhaft zu illustrieren.

Bei den Rezepturen 1 bis 6 handelt es sich um heißhergestellte W/O-Emulsionen, bei den Rezepturen 7 bis 9 um kalthergestellte W/O-Emulsionen. Die Herstellung erfolgte jeweils durch Einbringen der Wasserphase in die Ölphase und anschließendes Homogenisieren nach üblichen Methoden.

Nomenklatur gemäß INCI:

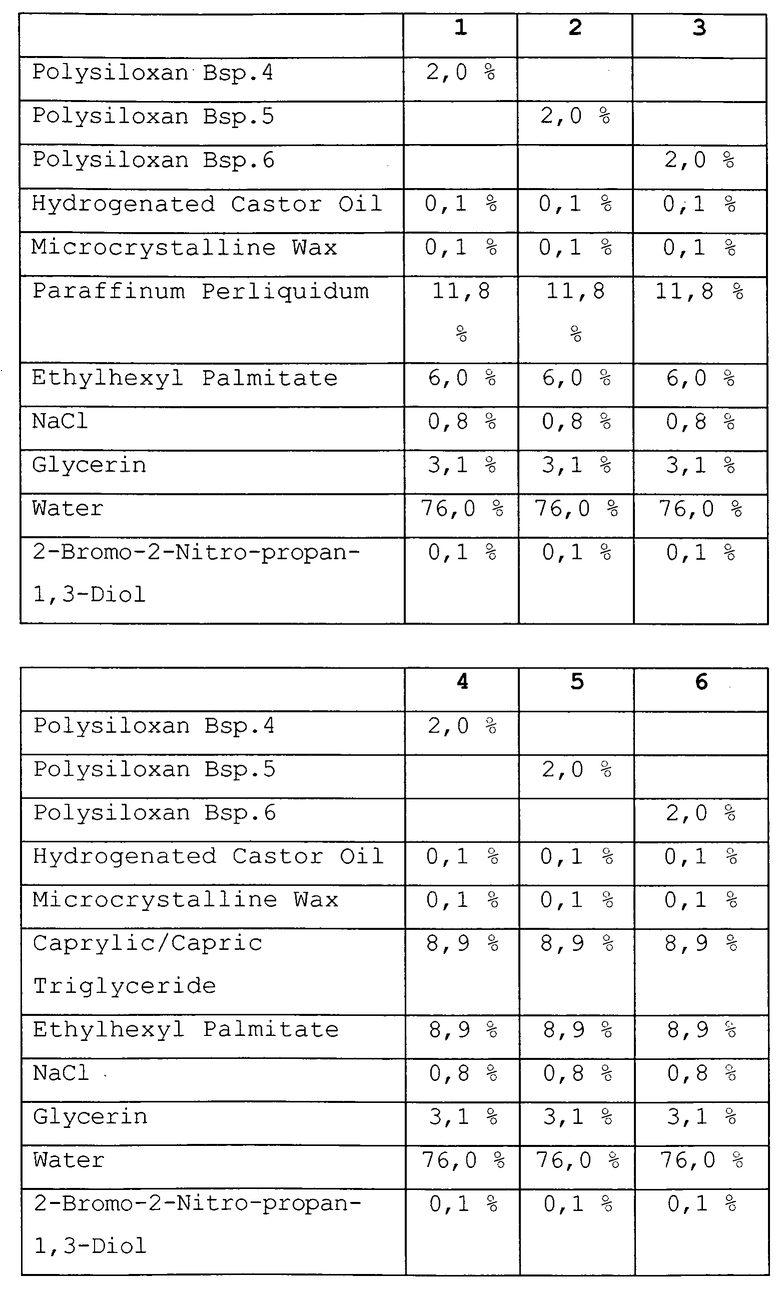

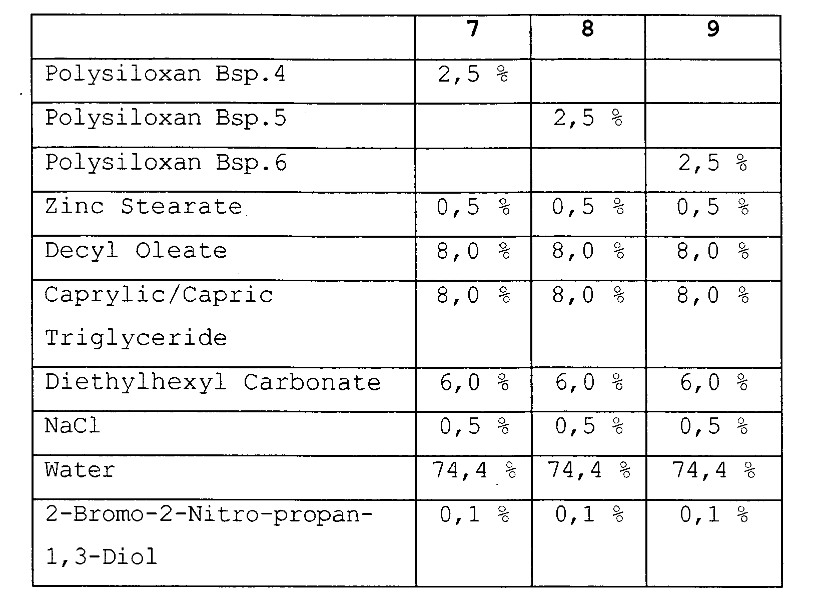

## Patentansprüche

1. Emulgator-Systeme, bestehend aus den modifizierten Siloxanen der allgemeinen Formel (I) 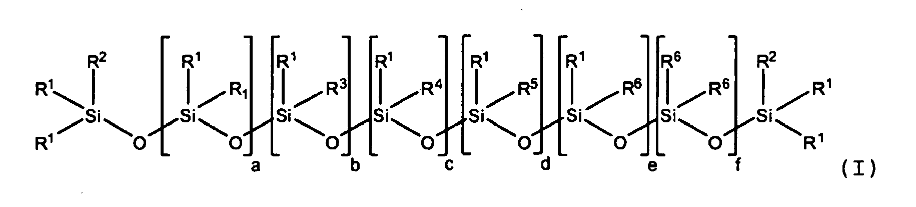 wobei
N a + b + c + d + e + f + 2 = 20 bis 250,
a 1 bis 230,
b 1 bis 100,
c 1 bis 100,
d 0 bis 50,
e 0 bis 10,
f 0 bis 10,
R¹ unabhängig voneinander gleich oder verschieden und ausgewählt aus der Gruppe enthaltend gesättigte oder ungesättigte, gegebenenfalls verzweigte Alkylgruppen mit 1 bis 30 C-Atomen, Alkarylreste mit 7 bis 30 C-Atomen, Arylreste mit 6 bis 30 C-Atomen,
R² unabhängig voneinander gleich oder verschieden und ausgewählt aus der Gruppe R¹, R³, R⁴, oder R⁵,
R³ unabhängig voneinander gleiche oder verschiedene Esterreste der allgemeinen Formel (Ia) 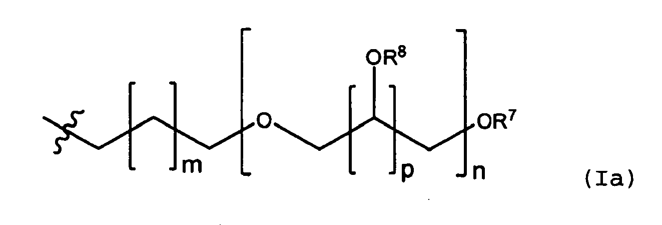 wobei
m 1 bis 4,
n 0 oder 1,
p 0 oder 1,
R⁷ die Acylreste einbasischer, gesättigter oder ungesättigter, linearer oder verzweigter Fettsäuren mit 6 bis 30 Kohlenstoffatomen,
R⁸ Wasserstoff oder R⁷
R⁴ die Reste der allgemeinen Formel (Ib) 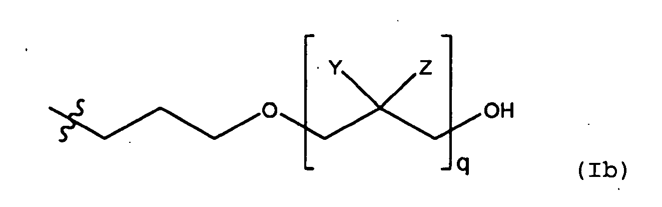 wobei
q 1 bis 20,
Y und Z unabhängig voneinander gleiche oder verschiedene Reste aus der Gruppe -H, -OH, -CH₃, -CH₂CH₃ oder -CH₂OH, sind, wobei mindestens ein Rest aus der Gruppe -OH oder -CH₂OH sein muss,
R⁵ unabhängig voneinander gesättigte oder ungesättigte, gegebenenfalls verzweigte Alkylgruppen mit 1 bis 30 C-Atomen oder Alkarylreste mit 7 bis 30 C-Atomen,
R⁶ unabhängig voneinander gleiche oder verschiedene Reste der allgemeinen Formel (Ic) 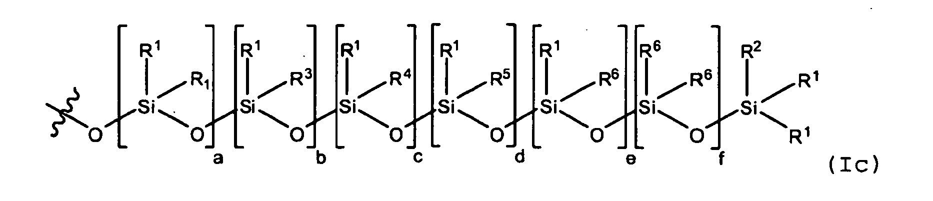 wobei a, b, c, d, e, f, R¹, R², R³, R⁴, R⁵ und R⁶ wie oben definiert sind,
und mindestens einem Co-Emulgator.

2. Emulgator-Systeme gemäß Anspruch 1
wobei
N a + b +c + d + e + f + 2 = 50 bis 150,
a 10 bis 130,
b 5 bis 30,
c 5 bis 30,
d 0,
e 0,
f 0,
R¹ Alkylgruppen mit 1 bis 4 C-Atomen,
R² R¹,
m 1 oder 4,
n 0 oder 1,
p 0 oder 1,
R⁷ die Acylreste einbasischer, gesättigter oder ungesättigter, linearer oder verzweigter Fettsäuren mit 8 bis 22 Kohlenstoffatomen,
R⁸ Wasserstoff,
q 1,
Y -OH und
Z -H,
sind.

3. Verwendung mindestens eines modifizierten Siloxans der allgemeinen Formel (I) wie in Anspruch 1 oder 2 beschrieben als Wasser-in-Öl-, Öl-in-Wasser- oder Wasser-in-Silicon-Emulgator oder als Dispergierhilfsmittel.

4. Wasser-in-Öl-Emulsionen, enthaltend mindestens eines modifiziertes Siloxan der allgemeinen Formel (I) wie in Anspruch 1 oder 2 beschrieben.

5. Öl-in-Wasser-Emulsion, enthaltend mindestens ein modifiziertes Siloxan der allgemeinen Formel (I) wie in Anspruch 1 oder 2 beschrieben.

6. Wasser-in-Silicon-Emulsionen, enthaltend mindestens ein modifiziertes Siloxan der allgemeinen Formel (I) wie in Anspruch 1 oder 2 beschrieben.

7. Dispersionen, enthaltend mindestens ein modifiziertes Siloxan der allgemeinen Formel (I) wie in Anspruch 1 oder 2 beschrieben.

8. Verwendung mindestens eines der Emulgator-Systeme gemäß Anspruch 1 oder 2 oder mindestens einer der Emulsionen gemäß den Ansprüchen 4 bis 6 oder mindestens einer der Dispersionen gemäß Anspruch 7 zur Herstellung kosmetischer, dermatologischer oder pharmazeutischer Formulierungen.

9. Kosmetische, dermatologische oder pharmazeutische Formulierungen enthaltend mindestens eines der Emulgator-Systeme gemäß Anspruch 1 oder 2 oder mindestens eine der Emulsionen gemäß den Ansprüchen 4 bis 6 oder mindestens eine der Dispersionen gemäß Anspruch 7.

10. Verwendung mindestens eines der Emulgator-Systeme gemäß Anspruch 1 oder 2 oder mindestens einer der Emulsionen gemäß den Ansprüchen 4 bis 6 oder mindestens einer der Dispersionen gemäß Anspruch 7 zur Herstellung von Pflege- und Reinigungsmitteln für Haushalt, Industrie, insbesondere für harte Oberflächen, Leder oder Textilien.

11. Pflege- und Reinigungsmittel für Haushalt, für harte Oberflächen, Leder oder Textilien enthaltend mindestens eines der Emulgator-Systeme gemäß Anspruch 1 oder 2 oder mindestens eine der Emulsionen gemäß den Ansprüchen 4 bis 6 oder mindestens eine der Dispersionen gemäß Anspruch 7.

## Claims

1. Emulsifier systems comprising the modified siloxanes of the general formula (I) 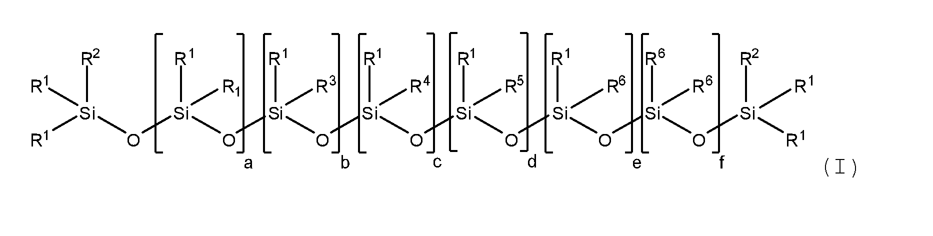 where
N is a + b + c + d + e + f + 2 = 20 to 250,
a is 1 to 230,
b is 1 to 100,
c is 1 to 100,
d is 0 to 50,
e is 0 to 10,
f is 0 to 10,
R¹ independently of the others is identical or different and selected from the group comprising saturated or unsaturated, optionally branched alkyl groups having 1 to 30 carbon atoms, alkaryl radicals having 7 to 30 carbon atoms, aryl radicals having 6 to 30 carbon atoms,
R² independently of the others is identical or different and selected from the group R¹, R³, R⁴ or R⁵,
R³ independently of the others is identical or different ester radicals of the general formula (Ia) 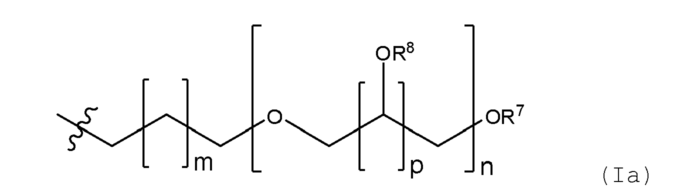 where
m is 1 to 4,
n is 0 or 1,
p is 0 or 1,
R⁷ is the acyl radicals of monobasic, saturated or unsaturated, linear or branched fatty acids having 6 to 30 carbon atoms,
R⁸ is hydrogen or R⁷,
R⁴ is the radicals of the general formula (Ib) 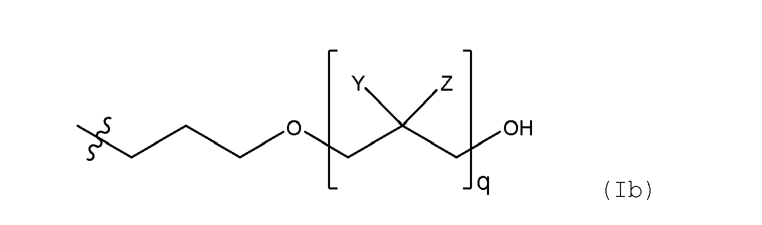 where
q is 1 to 20,
Y and Z, independently of one another, are identical or different radicals from the group -H, -OH, -CH₃, -CH₂CH₃ or -CH₂OH, where at least one radical must be from the group -OH or - CH₂OH,
R⁵ independently of the others is saturated or unsaturated, optionally branched alkyl groups having 1 to 30 carbon atoms or alkaryl radicals having 7 to 30 carbon atoms,
R⁶ independently of the others is identical or different radicals of the general formula (Ic) 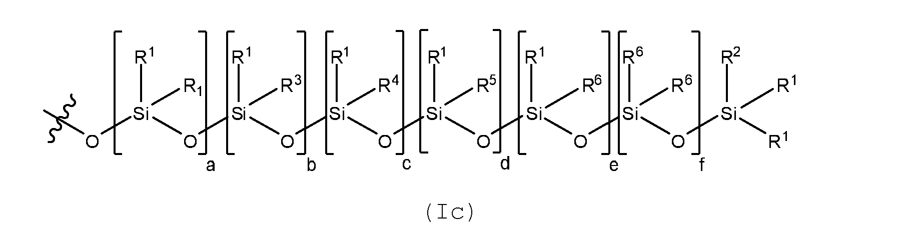 where a, b, c, d, e, f, R¹, R², R³, R⁴, R⁵ and R⁶ are as defined above,
and at least one coemulsifier.

2. Emulsifier systems according to Claim 1
where
N is a + b +c + d + e + f + 2 = 50 to 150,
a is 10 to 130,
b is 5 to 30,
c is 5 to 30,
d is 0,
e is 0,
f is 0,
R¹ is alkyl groups having 1 to 4 carbon atoms,
R² is R¹,
m is 1 or 4,
n is 0 or 1,
p is 0 or 1,
R⁷ is the acyl radicals of monobasic, saturated or unsaturated, linear or branched fatty acids having 8 to 22 carbon atoms,
R⁸ is hydrogen,
q is 1,
Y is -OH and
Z is -H.

3. Use of at least one modified siloxane of the general formula (I) as described in Claim 1 or 2 as water-in-oil, oil-in-water or water-in-silicone emulsifier or as dispersion auxiliary.

4. Water-in-oil emulsions comprising at least one modified siloxane of the general formula (I) as described in Claim 1 or 2.

5. Oil-in-water emulsion comprising at least one modified siloxane of the general formula (I) as described in Claim 1 or 2.

6. Water-in-silicone emulsions comprising at least one modified siloxane of the general formula (I) as described in Claim 1 or 2.

7. Dispersions comprising at least one modified siloxane of the general formula (I) as described in Claim 1 or 2.

8. Use of at least one of the emulsifier systems according to Claim 1 or 2 or at least one of the emulsions according to Claims 4 to 6 or at least one of the dispersions according to Claim 7 for producing cosmetic, dermatological or pharmaceutical formulations.

9. Cosmetic, dermatological or pharmaceutical formulations comprising at least one of the emulsifier systems according to Claim 1 or 2 or at least one of the emulsions according to Claims 4 to 6 or at least one of the dispersions according to Claim 7.

10. Use of at least one of the emulsifier systems according to Claim 1 or 2 or at least one of the emulsions according to Claims 4 to 6 or at least one of the dispersions according to Claim 7 for producing care and cleaning compositions for domestic use, industry, in particular for hard surfaces, leather or textiles.

11. Care and cleaning compositions for domestic use, for hard surfaces, leather or textiles comprising at least one of the emulsifier systems according to Claim 1 or 2 or at least one of the emulsions according to Claims 4 to 6 or at least one of the dispersions according to Claim 7.

## Revendications

1. Systèmes d'émulsifiants, constitués des siloxanes modifiés de formule générale (I) 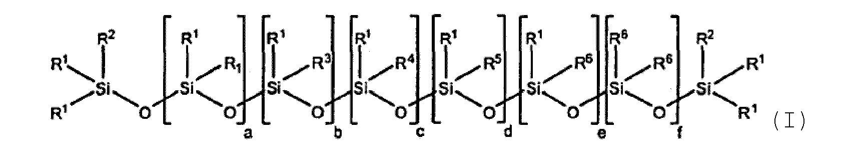 dans laquelle
la somme N a + b + c + d + e + f + 2 = 20 à 250,
a vaut de 1 à 230,
b vaut de 1 à 100,
c vaut de 1 à 100,
d vaut de 0 à 50,
e vaut de 0 à 10,
f vaut de 0 à 10,
R¹ indépendamment les uns des autres sont identiques ou différents et choisis dans l'ensemble contenant des groupes alkyle saturés ou insaturés, éventuellement ramifiés, ayant de 1 à 30 atomes de carbone, des radicaux alkaryle ayant de 7 à 30 atomes de carbone, des radicaux aryle ayant de 6 à 30 atomes de carbone,
R² indépendamment l'un de l'autre sont identiques ou différents et choisis dans l'ensemble constitué par R¹, R³, R⁴ et R⁵,
R³ indépendamment les uns des autres représentent des radicaux ester identiques ou différents de formule générale (Ia) 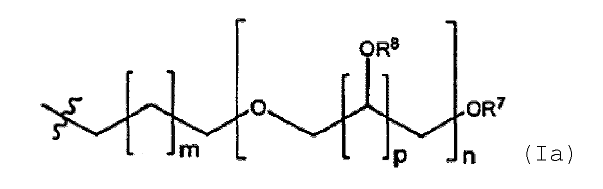 dans laquelle
m vaut de 1 à 4,
n vaut 0 ou 1,
p vaut 0 ou 1,
R⁷ représente les restes acyle d'acides gras monobasiques, saturés ou insaturés, linéaires ou ramifiés, ayant de 6 à 30 atomes de carbone,
R⁸ représente un atome d'hydrogène ou R⁷
R⁴ représente les radicaux de formule (Ib) 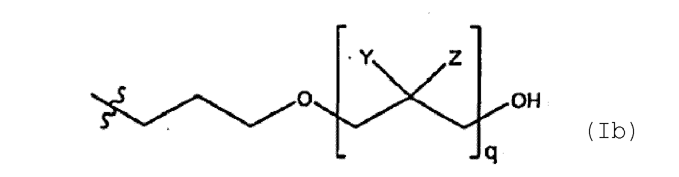 dans laquelle
q vaut de 1 à 20,
Y et Z indépendamment les uns des autres représentent des radicaux identiques ou différents choisis dans l'ensemble constitué par -H, -OH, -CH₃, -CH₂CH₃ et -CH₃OH, au moins un radical devant être choisi parmi -OH et -CH₂OH,
R⁵ indépendamment les uns des autres représentent des groupes alkyle saturés ou insaturés, éventuellement ramifiés, ayant de 1 à 30 atomes de carbone ou des radicaux alkaryle ayant de 7 à 30 atomes de carbone,
R⁶ indépendamment les uns des autres représentent des radicaux identiques ou différents, de formule générale (Ic) 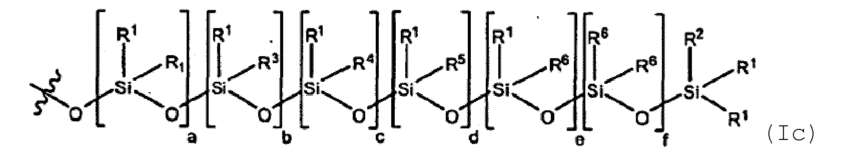 a, b, c, d, e, f, R¹, R², R³, R⁴, R et R étant tels que définis plus haut,
et d'au moins un co-émulsifiant.

2. Systèmes d'émulsifiants selon la revendication 1
dans lesquels
la somme N a + b + c + d + e + f + 2 = 50 à 150,
a vaut de 10 à 130,
b vaut de 5 à 30,
c vaut de 5 à 30,
d est 0,
e est 0,
f est 0,
R¹ représente des groupes alkyle ayant de 1 à 4 atomes de carbone,
R² est R¹,
m vaut 1 ou 4,
n vaut 0 ou 1,
p vaut 0 ou 1,
R⁷ représente les restes acyle d'acides gras monobasiques, saturés ou insaturés, linéaires ou ramifiés ayant de 8 à 22 atomes de carbone,
R⁸ représente un atome d'hydrogène,
q est 1,
Y est -OH et
Z est -H.

3. Utilisation d'au moins un siloxane modifié de formule générale (I) tel que décrit dans la revendication 1 ou 2, en tant qu'émulsifiant eau-dans-huile, huile-dans-eau ou eau-dans-silicone, ou en tant qu'adjuvant de dispersion.

4. Émulsions eau-dans-huile, contenant au moins un siloxane modifié de formule générale (I) telle que décrite dans la revendication 1 ou 2.

5. Émulsion huile-dans-eau, contenant au moins un siloxane modifié de formule générale (I) telle que décrite dans la revendication 1 ou 2.

6. Émulsions eau-dans-silicone, contenant au moins un siloxane modifié de formule générale (I) telle que décrite dans la revendication 1 ou 2.

7. Dispersions, contenant au moins un siloxane modifié de formule générale (I) telle que décrite dans la revendication 1 ou 2.

8. Utilisation d'au moins l'un des systèmes d'émulsifiants selon la revendication 1 ou 2 ou d'au moins l'une des émulsions selon les revendications 4 à 6 ou d'au moins l'une des dispersions selon la revendication 7 pour la fabrication de compositions cosmétiques, dermatologiques ou pharmaceutiques.

9. Compositions cosmétiques, dermatologiques ou pharmaceutiques contenant au moins l'un des systèmes d'émulsifiants selon la revendication 1 ou 2 ou au moins l'une des émulsions selon les revendications 4 à 6 ou au moins l'une des dispersions selon la revendication 7.

10. Utilisation d'au moins l'un des systèmes d'émulsifiants selon la revendication 1 ou 2 ou d'au moins l'une des émulsions selon les revendications 4 à 6 ou d'au moins l'une des dispersions selon la revendication 7 pour la fabrication de produits d'entretien et de nettoyage pour la maison, l'industrie, en particulier pour surfaces dures, cuir ou textiles.

11. Produits d'entretien et de nettoyage pour la maison, pour surfaces dures, cuir ou textiles, contenant au moins l'un des systèmes d'émulsifiants selon la revendication 1 ou 2 ou au moins l'une des émulsions selon les revendications 4 à 6 ou au moins l'une des dispersions selon la revendication 7.
